# EUROPÄISCHE PATENTSCHRIFT

(11) **EP 3 278 756 B1**
(45) Veröffentlichungstag und Bekanntmachung des Hinweises auf die Patenterteilung: **13.07.2022**
(21) Anmeldenummer: 17184296.6
(22) Anmeldetag: 01.08.2017
(51) Int. Cl.: A61B 34/00, A61B 17/00, A61B 34/20, A61B 90/50

(54) **SYSTEM UND VERFAHREN ZUM ÄNDERN DES BETRIEBSZUSTANDES EINER VORRICHTUNG**
SYSTEM AND METHOD FOR CHANGING THE OPERATIONAL STATE OF A DEVICE
SYSTÈME ET PROCÉDÉ DE MODIFICATION DE MODE DE FONCTIONNEMENT D'UN DISPOSITIF

(30) Priorität: 05.08.2016 DE 102016114601
(43) Veröffentlichungstag der Anmeldung: 07.02.2018
(73) Patentinhaber: Aesculap AG, 78532 Tuttlingen (DE)
(72) Erfinder: Maas, Allan, 78467 Konstanz (DE); Wehrle, Christian, 78269 Volkertshausen (DE); Beger, Jens, 78532 Tuttlingen (DE); Kozak, Josef, 78532 Tuttlingen (DE)
(74) Vertreter: Hoeger, Stellrecht & Partner Patentanwälte mbB

(56) Entgegenhaltungen:
- WO-A1-2015/164402
- US-A- 5 091 926
- US-A1- 2016 000 514

## Beschreibung

Die Erfindung betrifft ein System zum Ändern des Betriebszustandes mindestens einer elektrisch betriebenen Vorrichtung, insbesondere einer medizintechnischen Vorrichtung.

Offenbart ist ferner ein Verfahren zum Ändern des Betriebszustandes mindestens einer elektrisch betriebenen Vorrichtung, insbesondere einer medizintechnischen Vorrichtung.

Die Vorrichtung kann insbesondere eine medizintechnische Vorrichtung sein, und die vorliegende Erfindung wird nachfolgend am Beispiel einer derartigen medizintechnischen Vorrichtung erläutert, ist jedoch nicht auf dieses Anwendungsgebiet beschränkt.

Während einer Operation können von einem Operateur und dessen Assistenten eine Vielzahl unterschiedlicher elektrisch betriebener Vorrichtungen zum Einsatz kommen. Wünschenswert wäre es, die Bedienung elektrisch betriebener Vorrichtungen zu vereinfachen.

Die US 2016/0000514 A1 beschreibt ein chirurgisches Werkzeug und eine Anzeigeeinrichtung zum Durchführen chirurgischer Verfahren, wobei ein am Kopf tragbares Gerät vorgesehen ist, das einen Rahmen zum Positionieren einer optischen Anzeige im Sichtfeld eines Betrachters aufweist. Eine Steuereinheit ist an einem Abschnitt des Rahmens angebracht und dazu ausgebildet, an der optischen Anzeige ein computergeneriertes Bild zu erzeugen. Eine optische Anzeige, umfassend eine aktive Seite zum Projizieren eines computergenerierten Bildes, steht elektrisch mit der Steuereinheit in Verbindung und ist an der Steuereinheit derart angebracht, so dass die aktive Seite im Sichtfeld sichtbar ist.

Die WO 2015/164402 A1 beschreibt ein intra-operatives medizintechnisches Bildbetrachtungssystem, das es einem Chirurgen erlaubt, eine Betrachtungsperspektive auf den Patienten aufrechtzuerhalten, während optische Bilder im laufenden Betrieb aufgerufen werden. Eine digitale Bildquelle enthält mindestens eine Bilddatei, die charakteristisch für ein anatomisches oder pathologisches Merkmal des Patienten ist. Eine Anzeige wird vom Operateur getragen oder ist während der Operation zwischen dem Operateur und dem Patienten angeordnet. Die Anzeige ist selektiv transparent und stellt dem Operateur ein Bild dar, das aus der Bilddatei abgeleitet ist. Eine Bildsteuereinheit liest die Bilddatei von der Bildquelle und steuert die Anzeige derart, so dass zumindest ein Abschnitt des Bildes angezeigt und vom Operateur nach dessen Willen modifiziert werden kann. Eine Mehrzahl von Peripheriegeräten sind jeweils dazu ausgestaltet, einen Bildsteuereingang vom Operateur zu empfangen und, als Antwort darauf, ein Bildsteuersignal zu erzeugen.

Aufgabe der vorliegenden Erfindung ist es, ein System zum Ändern eines Betriebszustandes mindestens einer elektrisch betriebenen Vorrichtung bereitzustellen, das eine benutzerfreundliche Handhabung der Vorrichtung ermöglicht.

Diese Aufgabe wird durch ein erfindungsgemäßes System zum Ändern des Betriebszustandes mindestens einer elektrisch betriebenen Vorrichtung mit den Merkmalen von Anspruch 1 gelöst.

Das System umfasst eine Erfassungseinrichtung an einer Trageinrichtung, die am Körper einer Bedienperson tragbar und insbesondere lösbar halterbar ist. "Am Körper" schließt vorliegend eine Kleidung der Bedienperson oder einen anderweitig am Körper befestigten Gegenstand mit ein. Die Bedienperson kann sich derart bewegen, dass die Erfassungseinrichtung auf das mindestens eine Objekt ausgerichtet wird. Das Objekt kann, wenn im Erfassungsbereich der Erfassungseinrichtung angeordnet, von dieser erfasst und identifiziert werden. Der mindestens einen Vorrichtung, die mit dem mindestens einen Objekt verknüpft ist oder die das mindestens eine Objekt bildet, kann ein Signal übermittelt werden, und die Vorrichtung kann vom ersten Zustand in den zweiten Zustand überführt werden. Auf diese Weise ist eine benutzerfreundliche Möglichkeit für die Bedienperson vorhanden, auf die mindestens eine Vorrichtung einzuwirken. Dabei besteht insbesondere die Möglichkeit, das mindestens eine Objekt kontaktlos zu erfassen. Eine kontaktlose Erfassung ist beispielsweise durch eine von der Erfassungseinrichtung umfasste oder gebildete Kamera möglich.

Das Signal kann der Vorrichtung von der Erfassungseinrichtung direkt oder indirekt, beispielsweise über eine Steuereinrichtung, übermittelbar sein.

Das System kann eine Mehrzahl von Vorrichtungen und Objekten umfassen. Jeder Vorrichtung kann/können ein oder mehrere Objekt zugeordnet sein. Jedes Objekt kann einer oder mehreren Vorrichtungen zugeordnet sein.

Die Handhabung des Systems wird dadurch verbessert, dass die mindestens eine Vorrichtung nur dann vom ersten Zustand in den zweiten Zustand oder umgekehrt überführt wird, wenn das mindestens eine Objekt für eine vorgegebene oder vorgebbare Zeitdauer von der Erfassungseinrichtung erfasst wird. Beispielsweise ist eine Zeitdauer von wenigen Sekunden vorgesehen, damit eine Zustandsänderung an der mindestens einen Vorrichtung nicht jedes Mal dann eintritt, wenn der Erfassungsbereich das mindestens eine Objekt auch nur für sehr kurze Zeit überstreicht. Beispielsweise kann erforderlich sein, das mindestens eine Objekt über die vorgegebene oder vorgebbare Zeitdauer zu identifizieren.

Als besonders vorteilhaft erweist sich das System, wenn die mindestens eine Vorrichtung batteriebetrieben ist. Beispielsweise gibt dies die Möglichkeit, die batteriebetriebene Vorrichtung durch Überführen vom ersten in den zweiten Zustand bei Bedarf einzuschalten und vorzugsweise nach Beendigung des Einsatzes wieder vom zweiten Zustand in den ersten Zustand zu überführen und abzuschalten. Die Laufzeit batteriebetriebener Vorrichtungen kann dadurch gesteigert werden. Es ist eine bedarfsabhängige, benutzerfreundliche Aktivierung batteriebetriebener Vorrichtungen möglich.

Der erste Zustand kann insbesondere ein Ausschaltzustand der mindestens einen Vorrichtung sein und der zweite Zustand mindestens ein Einschaltzustand der mindestens einen Vorrichtung oder umgekehrt. Dies erweist sich insbesondere in Kombination mit batteriebetriebenen Vorrichtungen als vorteilhaft.

Alternativ ist es von Vorteil, wenn der erste Zustand ein erster Betriebszustand der mindestens einen Vorrichtung ist und der zweite Zustand ein zweiter Betriebszustand der mindestens einen Vorrichtung in einem eingeschalteten Zustand derselben. Der erste und der zweite Zustand sind beispielsweise unterschiedliche Betriebsstufen oder Betriebsmodi der mindestens einen Vorrichtung. Beispielsweise ist der erste Zustand ein Stand-by-Zustand, den die mindestens eine Vorrichtung energiesparend einnehmen werden kann. Der zweite Zustand kann ein aktivierter Betriebszustand sein.

Von Vorteil ist es, wenn die Erfassungseinrichtung optisch ausgestaltet ist und eine Kamera zum Erfassen des mindestens einen Objektes ist oder umfasst. Dies erlaubt eine konstruktiv einfache Ausgestaltung des Systems und insbesondere eine berührungslose Erfassung des mindestens einen Objektes im Erfassungsbereich. Dabei ist vorzugsweise eine Erfassung des mindestens einen Objektes über einen größeren Abstandsbereich von der Erfassungseinrichtung möglich, beispielsweise von einigen zehn Zentimetern bis zu einigen Metern. Das Objekt kann identifiziert werden, und der entsprechenden Vorrichtung kann ein Signal übermittelt werden.

Vorzugsweise steht die Erfassungseinrichtung kabellos mit der mindestens einen Vorrichtung in Wirkverbindung, insbesondere über eine Funkverbindung. Die Bedienperson wird dadurch in ihrer Bewegungsfreiheit nicht eingeschränkt. Beispielsweise wird ein Signal von der Erfassungseinrichtung mittels Bluetooth (insbesondere Bluetooth Low Energy) an die mindestens eine Vorrichtung übertragen.

Vorteilhafterweise ist an der Trageinrichtung mindestens eine Batterie zur Energieversorgung der Erfassungseinrichtung gehalten.

Günstig ist es, wenn die Trageinrichtung am Kopf der Bedienperson anordenbar ist. Durch die Anordnung am Kopf werden Bewegungen des Kopfes, die zu einer Bewegung der Augen der Bedienperson und damit des Sichtfeldes der Bedienperson führen, in eine Bewegung der Trageinrichtung mit der Erfassungseinrichtung überführt. Die Erfassungseinrichtung kann dadurch auf konstruktiv einfache Weise und benutzerfreundlich möglichst gezielt auf das mindestens eine Objekt ausgerichtet werden.

Bei einer vorteilhaften Ausführungsform des Systems ist oder umfasst die Trageinrichtung eine Brille oder ein Brillengestell. Darunter kann vorliegend insbesondere verstanden werden, dass die Trageinrichtung nach Art einer Brille oder eines Brillengestelles getragen werden kann. Nicht erforderlich ist, dass Brillengläser, geschliffen oder ungeschliffen, vorhanden sind. Ausreichend ist beispielsweise ein Gestell zum An- oder Auflegen an den Ohren und der Nase.

Bei einer besonders vorteilhaften Ausführungsform ist die Trageinrichtung mit der Erfassungseinrichtung als Datenbrille ausgestaltet. Diese weist vorzugsweise, darauf wird nachfolgend noch eingegangen, eine Anzeigeeinrichtung auf. Eine mit der Erfassungseinrichtung und/oder der Anzeigeeinrichtung gekoppelte Steuereinrichtung kann ebenfalls Bestandteil der Datenbrille sein. Die Anzeigeeinrichtung und die Steuereinrichtung können an der Brille oder am Brillengestell gehalten sein.

Bei einer andersartigen vorteilhaften Ausführungsform des Systems ist oder Umfasst die Trageinrichtung ein Stirnband oder eine Haube.

Es kann vorgesehen sein, dass die Vorrichtung den zweiten Zustand solange einnimmt, wie das mindestens eine Objekt von der Erfassungseinrichtung erfasst wird, und anderenfalls den ersten Zustand einnimmt.

Alternativ kann vorgesehen sein, dass die Vorrichtung nach der Einnahme des zweiten Zustandes in diesem verbleibt und vom zweiten Zustand in den ersten Zustand überführbar ist, wenn das mindestens eine Objekt von der Erfassungseinrichtung erneut erfasst und der mindestens einen Vorrichtung ein diesbezügliches Signal übermittelt wird.

Alternativ kann vorgesehen sein, dass die mindestens eine Vorrichtung für eine vorgegebene oder vorgebbare Zeitdauer den zweiten Zustand einnimmt und anschließend in den ersten Zustand zurückkehrt.

Als vorteilhaft erweist es sich, wenn das mindestens eine Objekt ein Markierelement ist. Das Markierelement kann zum Beispiel ein Aufkleber oder eine Plakette sein oder umfassen. Am Markierelement kann ein Code hinterlegt sein, beispielsweise ein Matrixcode. Das Markierelement kann ein codierter Marker oder ein AR-Marker sein.

Das Markierelement kann an der mindestens einen Vorrichtung festgelegt sein.

Bei einer andersartigen vorteilhaften Ausführungsform ist es günstig, wenn als das mindestens eine Objekt die mindestens eine Vorrichtung von der Erfassungseinrichtung optisch erfassbar ist zum Erkennen der mindestens einen Vorrichtung anhand von deren charakteristischen Merkmalen, insbesondere Form und/oder Farbe.

In der Erfassungseinrichtung können Bildverarbeitungsalgorithmen hinterlegt sein zum Analysieren von Aufnahmen, die von der Erfassungseinrichtung erstellt werden. Die Aufnahmen werden dahingehend analysiert, ob das mindestens eine Objekt identifiziert werden kann, zum Beispiel durch charakteristisehe Merkmale der mindestens einen Vorrichtung oder durch charakteristische Merkmale des vorstehend genannten Markierelementes.

Eine vorteilhafte Ausführungsform des Systems sieht vor, dass eine Vorrichtung mit mindestens einem antreibbaren chirurgischen Bearbeitungswerkzeug vorgesehen ist, wobei der erste Zustand und der zweite Zustand mit dem Antrieb des Bearbeitungswerkzeuges verknüpft sind. Dies gibt die Möglichkeit, handhabungsfreundlich auf den Antrieb des Bearbeitungswerkzeuges einzuwirken. Vorteilhafterweise ist die Vorrichtung batteriebetrieben. Das Bearbeitungswerkzeug ist zum Beispiel ein chirurgisches Bohrwerkzeug oder ein chirurgisches Sägewerkzeug.

Alternativ oder ergänzend ist es günstig, wenn eine Vorrichtung mit mindestens einem Leuchtelement vorgesehen ist, wobei der erste Zustand und der zweite Zustand mit dem Leuchtzustand des Leuchtelementes verknüpft sind. Dies gibt die Möglichkeit, handhabungsfreundlich auf den Leuchtzustand des Leuchtelementes einzuwirken. Das Leuchtelement kann zum Beispiel Bestandteil einer endoskopischen Leuchteinrichtung sein. Denkbar ist auch, dass das Leuchtelement Bestandteil eines chirurgischen Navigationssystems ist, um von diesem erfassbare medizinische Markierelemente zu beleuchten. Die Vorrichtung kann batteriebetrieben sein.

Alternativ oder ergänzend ist es günstig, wenn eine bildgebende Vorrichtung mit mindestens einer Kamera vorgesehen ist, wobei der erste Zustand und der zweite Zustand mit einer Bereitstellung eines Bildes der Kamera verknüpft sind. Dies gibt auf handhabungsfreundliche Weise die Möglichkeit, auf die Bereitstellung eines Bildes einzuwirken. Beispielsweise wird nur im ersten oder zweiten Zustand ein Bild bereitgestellt, im jeweils anderen Zustand nicht. Die Vorrichtung kann batteriebetrieben sein.

Die bildgebende Vorrichtung ist beispielsweise eine endoskopische Vorrichtung. Die bildgebende Vorrichtung kann auch ein chirurgisches Navigationssystem sein, zum Beispiel mit einer Stereokamera, deren Bilder zur Ermittlung von Positionsdaten medizinische Markierelemente herangezogen werden können.

Günstig ist es, wenn die mindestens eine Vorrichtung eine Anzeigeeinrichtung mit einer Bildanzeige aufweist, an der ein Bildinhalt betreffend den Betrieb der mindestens einen Vorrichtung darstellbar ist. Hierbei kann insbesondere vorgesehen sein, dass an der Bildanzeige ein Bildinhalt abhängig vom ersten Zustand und/oder zweiten Zustand darstellbar ist. Dies schließt die Möglichkeit mit ein, dass in einem der Zustände die Darstellung eines Bildinhaltes entfällt, die Anzeigeeinrichtung also nichts darstellt.

Alternativ oder ergänzend kann vorgesehen sein, dass als Vorrichtung eine Anzeigeeinrichtung mit einer Bildanzeige vorgesehen ist, wobei der erste Zustand und der zweite Zustand mit einem Bildinhalt der Bildanzeige verknüpft sind, einschließlich der Möglichkeit, dass in einem Zustand die Darstellung eines Bildinhaltes entfallen kann. Dies gibt auf handhabungsfreundliche Weise die Möglichkeit, auf die Anzeigeeinrichtung einzuwirken. Die Anzeigeeinrichtung kann batteriebetrieben sein.

Bei den in den beiden letzten Absätzen genannten vorteilhaften Ausführungsformen kann günstigerweise vorgesehen sein, dass die Anzeigeeinrichtung an der Trageinrichtung gehalten ist. Besonders günstig ist es, wenn eine Datenbrille mit Anzeigeeinrichtung zur Einblendung des Bildinhaltes für die Bedienperson vorhanden ist. Die bereits vorstehend erwähnte Datenbrille kann dementsprechend mit einer Anzeigeeinrichtung versehen sein. Die Anzeigeeinrichtung kann der Bedienperson einen Bildinhalt darstellen, der vorzugsweise mit dem ersten Zustand und dem zweiten Zustand verknüpft ist und dabei vom jeweiligen Zustand abhängen kann.

Die Erfindung erlaubt es bei der zuletzt erwähnten vorteilhaften Ausführungsform beispielsweise, für den Benutzer eine Art "virtuellen Monitor" bereitzustellen. Beispielsweise ist ein Objekt in Gestalt eines Markierelementes an einer Wand, Stellfläche, Tischfläche oder anderweitig angeordnet. Durch Ausrichten der an der Datenbrille angeordneten Erfassungseinrichtung wird das Objekt erfasst und identifiziert. An der Anzeigeeinrichtung der Datenbrille kann ein Bildinhalt dargestellt werden, der für den Benutzer als gewissermaßen am "virtuellen Monitor" gezeigt angesehen werden kann.

Die eingangs genannte Aufgabe wird ferner durch ein Verfahren zum Ändern des Betriebszustandes mindestens einer elektrisch betriebenen Vorrichtung bei einem System der vorstehend genannten Art gelöst, wobei die Erfassungseinrichtung durch Bewegung der Bedienperson auf das mindestens eine Objekt ausgerichtet und das mindestens eine Objekt in einem Erfassungsbereich der Erfassungseinrichtung positioniert und von dieser erfasst und identifiziert wird, wobei von der Erfassungseinrichtung bei identifiziertem Objekt an die mindestens eine Vorrichtung ein diesbezügliches Signal derart übermittelt wird, dass die mindestens eine Vorrichtung vom ersten Zustand in den zweiten Zustand überführt wird, wobei die mindestens eine Vorrichtung nur dann vom ersten Zustand in den zweiten Zustand oder umgekehrt überführt wird, wenn das mindestens eine Objekt für eine vorgegebene oder vorgebbare Zeitdauer von der Erfassungseinrichtung erfasst wird.

Die Vorteile, die bereits im Zusammenhang mit der Erläuterung des erfindungsgemäßen Systems erwähnt wurden, können unter Ausübung des Verfahrens ebenfalls erzielt werden. Diesbezüglich kann auf die voranstehenden Ausführungen verwiesen werden.

Vorteilhafte Ausführungsbeispiele des Verfahrens ergeben sich durch vorteilhafte Ausführungsformen des erfindungsgemäßen Systems.

Die nachfolgende Beschreibung bevorzugter Ausführungsformen der Erfindung dient im Zusammenhang mit der Zeichnung der näheren Erläuterung der Erfindung. Die erfindungsgemäßen Systeme erlauben die Durchführung des offenbarten Verfahrens. Es zeigen:
- Figur 1:: eine erste bevorzugte Ausführungsform eines erfindungsgemäßen Systems in Anwendung an einem Patienten mit einer medizintechnischen Vorrichtung;
- Figur 2:: eine Darstellung entsprechend Figur 1, wobei die Vorrichtung einen unterschiedlichen Betriebszustand einnimmt;
- Figur 3:: eine zweite bevorzugte Ausführungsform eines erfindungsgemäßen Systems in Anwendung durch einen Operateur an einem Patienten mit einer bildgebenden, endoskopischen Vorrichtung;
- Figur 4:: eine schematische Teildarstellung des Systems, die die Darstellung eines Bildinhalts an einer Datenbrille des Systems zeigt; und

Figur 5:eine weitere bevorzugte Ausführungsform eines erfindungsgemäßen Systems mit einer Vorrichtung in Gestalt eines Navigationssystems.

Die Erfindung wird nachfolgend am Beispiel von Systemen mit medizintechnischen Vorrichtungen beschrieben. Jedoch ist die Erfindung wie bereits eingangs erwähnt nicht auf die Anwendung im medizinischen Umfeld beschränkt.

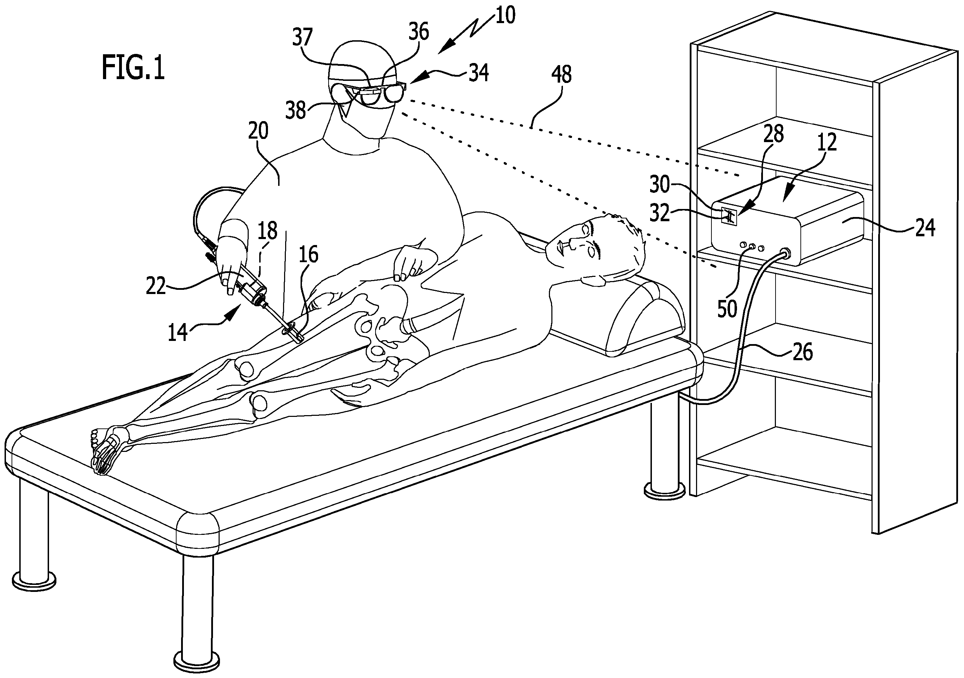

Figur 1 zeigt eine mit dem Bezugszeichen 10 belegte vorteilhafte Ausführungsform eines erfindungsgemäßen Systems. Das System 10 umfasst eine medizintechnische Vorrichtung 12. Die Vorrichtung 12 ist vorliegend batteriebetrieben.

Die Vorrichtung 12 kann ein chirurgisches Bearbeitungswerkzeug 14 aufweisen, das zum Beispiel einen chirurgischen Bohrer 16 umfasst. Es kann ein Antrieb 18 für den Bohrer 16 vorgesehen sein, der zum Beispiel in einem von einem Operateur 20 handhaltbaren Griffelement 22 angeordnet ist.

Der Operateur 20 wird nachfolgend im Sinne einer "Bedienperson" der vorstehenden Erläuterungen verstanden.

Weiter kann die Vorrichtung 12 ein Steuergerät 24 umfassen, das mit dem Bearbeitungswerkzeug 14 über eine elektrische Leitung 26 gekoppelt ist. Im Steuergerät 24 kann mindestens eine in der Zeichnung nicht dargestellte Batterie zum Bereitstellen elektrischer Energie für die Vorrichtung 12 angeordnet sein.

Der Vorrichtung 12 ist ein Objekt 28 zugeordnet, wobei insbesondere eine eineindeutige Zuordnung des Objektes 28 zur Vorrichtung 12 besteht. Bei Vorhandensein weiterer, in der Zeichnung nicht dargestellter Vorrichtungen kann mit einem jeweiligen weiteren Objekt eine eineindeutige Beziehung bestehen.

Das Objekt 28 ist im vorliegenden Fall ein Markierelement 30 mit einem Code 32. Das Markierelement kann zum Beispiel ein AR-Marker oder ein codierter Marker sein und einen Matrixcode aufweisen. Das Markierelement 30 ist zum Beispiel in Gestalt eines Aufklebers ausgestaltet und an der Vorrichtung 12 festgelegt.

Das System 10 umfasst außerdem eine Datenbrille 34 mit einem Brillengestell 36. Das Brillengestell 36 bildet eine Trageinrichtung 37. Brillengläser können vorhanden sein, dies ist jedoch nicht erforderlich.

Die Datenbrille 34 kann über die Trageinrichtung 37 vom Operateur 20 in an sich bekannter Weise am Kopf getragen werden, auf den Ohren und der Nase aufliegend. Bei einer Bewegung des Kopfes wird die Datenbrille 34 mitbewegt. Richtet der Operateur 20 seinen Kopf auf ein Ziel aus, beispielsweise das Objekt 28, ist die Datenbrille 34 ebenfalls auf das Objekt 28 ausgerichtet.

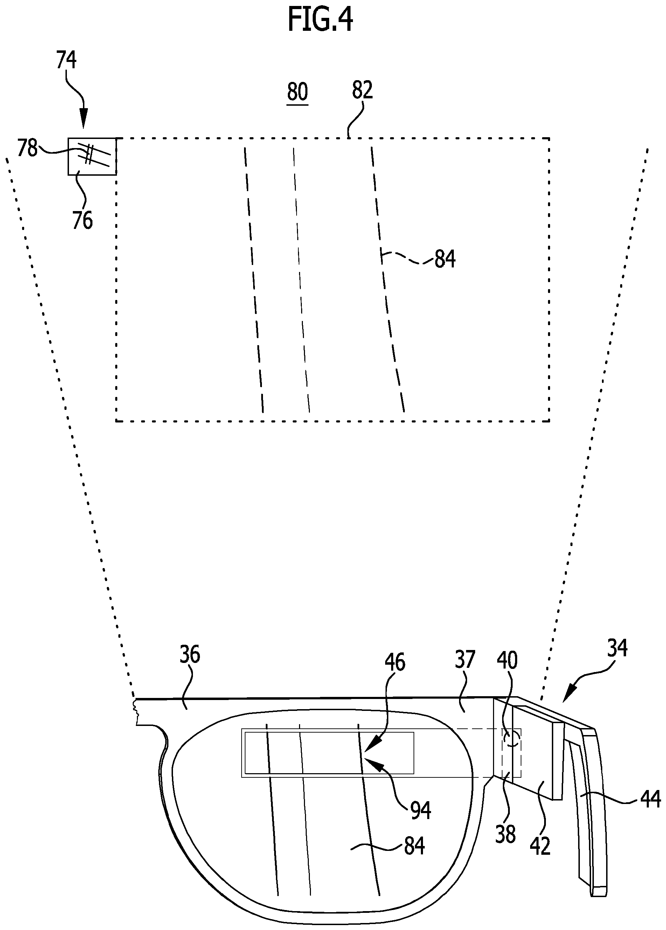

Die Figur 4 zeigt abschnittsweise eine vergrößerte Darstellung der Datenbrille 34. Die darin gezeigte Datenbrille 34 kommt bei allen hier erläuterten erfindungsgemäßen Systemen zum Einsatz.

Am Brillengestell 36 ist eine Erfassungseinrichtung 38 gehalten, die optisch ausgestaltet ist und eine Kamera 40 umfasst. Weiter kann am Brillengestell 36 eine Steuereinrichtung 42 gehalten sein. Die Steuereinrichtung 42 kann mit der Erfassungseinrichtung 38 gekoppelt oder in diese integriert sein, es kann also eine kombinierte Erfassungseinrichtung/Steuereinrichtung vorhanden sein. Nachfolgend wird nicht-limitierend davon ausgegangen, dass die Steuereinrichtung 42 von der Erfassungseinrichtung 38 getrennt gebildet und mit dieser gekoppelt ist.

Zur Energieversorgung der Datenbrille 34 kann am Brillengestell 36 eine wiederaufladbare Batterie 44 gehalten sein.

Das System 10 umfasst ferner eine Anzeigeeinrichtung 46 an der Datenbrille 34, die mit der Steuereinrichtung 42 gekoppelt ist. Die Anzeigeeinrichtung 46 umfasst eine Bildanzeige, an der mittels der Steuereinrichtung 42 Bildinhalte dargestellt werden. Im Zusammenhang mit der Ausführungsform des Systems 10 wird hierauf nicht weiter eingegangen, dies erfolgt bei der Erläuterung der weiteren vorteilhaften Ausführungsformen des erfindungsgemäßen Systems.

Die Erfassungseinrichtung 38 umfasst einen Erfassungsbereich 48, der durch ein Sichtfeld der Kamera 40 definiert ist. Die Kamera 40 ist so an der Datenbrille 34 angeordnet, dass bei ungezwungenem Geradeausblick des Operateurs 20 und bestimmungsgemäßer Tragweise der Datenbrille 34 die Kamera 40 mit dem Sichtfeld und damit der Erfassungsbereich 48 einen zentralen Abschnitt des Blickfeldes des Operateurs 20 bildet. Die Kamera 40 kann einen Abschnitt dessen erfassen, was der Operateur 20 sieht.

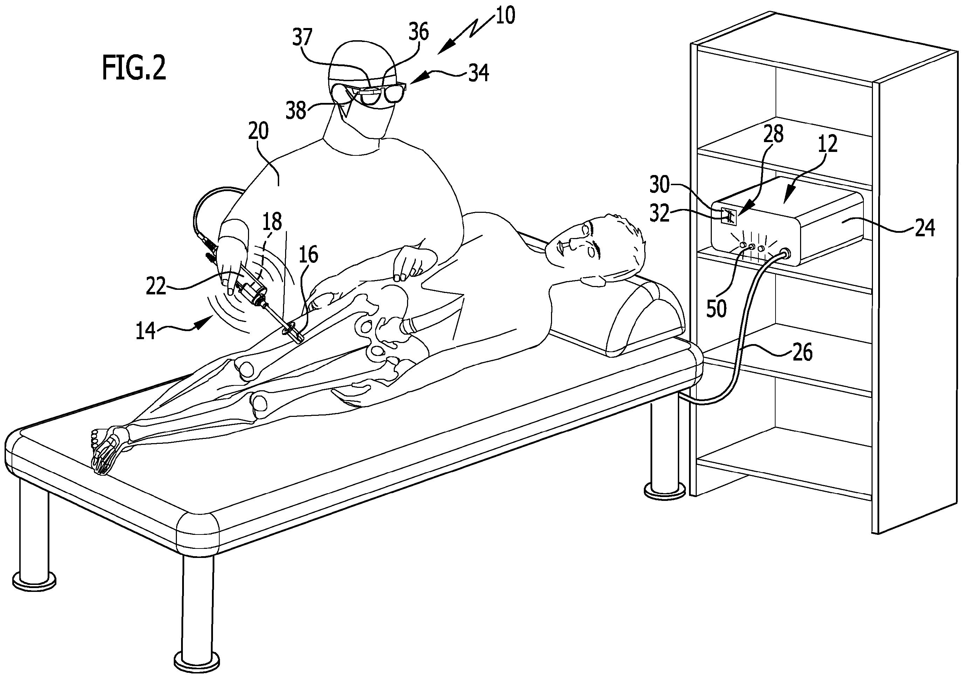

Die Funktionsweise des Systems 10 wird nachfolgend anhand der Figuren 1 und 2 erläutert.

Der Operateur 20 kann die Kamera 40 durch eine Bewegung des Kopfes und damit der Datenbrille 34 auf das Markierelement 30 ausrichten. Das Markierelement 30 ist dann im Erfassungsbereich 48 positioniert und wird von der Kamera 40 erfasst. Von der Erfassungseinrichtung 38 werden Aufnahmen der Kamera 40 dahingehend analysiert, ob Merkmale des Markierelementes 30 erfasst wurden.

Bejahendenfalls kann das Markierelement 30 identifiziert werden. Von der Erfassungseinrichtung 38 wird über die Steuereinrichtung 42 ein diesbezügliches Signal an die dem Markierelement 30 zugeordnete Vorrichtung 12 übermittelt. Beispielsweise wird das Signal an das Steuergerät 24 übermittelt.

Die Signalübermittlung erfolgt per Funk, beispielsweise mittels Bluetooth Low Energy-Standard.

Basierend auf dem ihr zugeführten Signal kann die Vorrichtung 12 von einem ersten Zustand in einen zweiten Zustand überführt werden. Vorliegend ist der erste Zustand ein Ausschaltzustand der Vorrichtung 12, bei dem insbesondere der Antrieb 18 deaktiviert ist.

Durch Erhalt des Signals wird die Vorrichtung 12 in den zweiten Zustand überführt, einen Einschaltzustand. Der Antrieb 18 wird aktiviert. Dies ist in Figur 2 durch angedeutete Schwingungen des Bearbeitungswerkzeugs 14 symbolisiert. Außerdem ist dies in Figur 2 durch aufleuchtende Anzeigeelemente 50 am Steuergerät 24 symbolisiert.

Günstig ist es, wenn das Überführen der Vorrichtung 12 vom Ausschaltzustand in den Einschaltzustand erst erfolgt, wenn das Markierelement 30 für eine vorgebbare oder vorgegebene Zeitdauer, beispielsweise wenige Sekunden, von der Kamera 40 erfasst und identifiziert wurde.

Die Vorrichtung 12 bleibt bei dem System 10 beispielsweise im zweiten Zustand so lange aktiviert, bis der Operateur 20 das Markierelement 30 erneut ins Visier nimmt und den Erfassungsbereich 48 auf das Markierelement 30 zu dessen Erfassung durch die Kamera 40 und Identifikation ausrichtet. Dadurch kann die Vorrichtung 12 von der Erfassungseinrichtung 38 über die Steuereinrichtung 42 abermals mit einem Signal über eine Änderung des Betriebszustandes informiert werden. Die Vorrichtung 12 wird vom Einschaltzustand in den Ausschaltzustand überführt.

Alternativ kann vorgesehen sein, dass die Vorrichtung 12 für eine vorgebbare oder vorgegebene Zeitdauer den zweiten Zustand einnimmt und anschließend in den ersten Zustand zurückkehrt.

Günstig ist insbesondere der Einsatz des Systems 10 bei batteriebetriebenen Vorrichtungen. Auf benutzerfreundliche und einfache Weise können diese vom Operateur 20 auch aus der Entfernung in Betrieb genommen und außer Betrieb gesetzt werden, so dass Energie der Batterie(n) gespart und die Laufzeit der Vorrichtungen gesteigert werden kann.

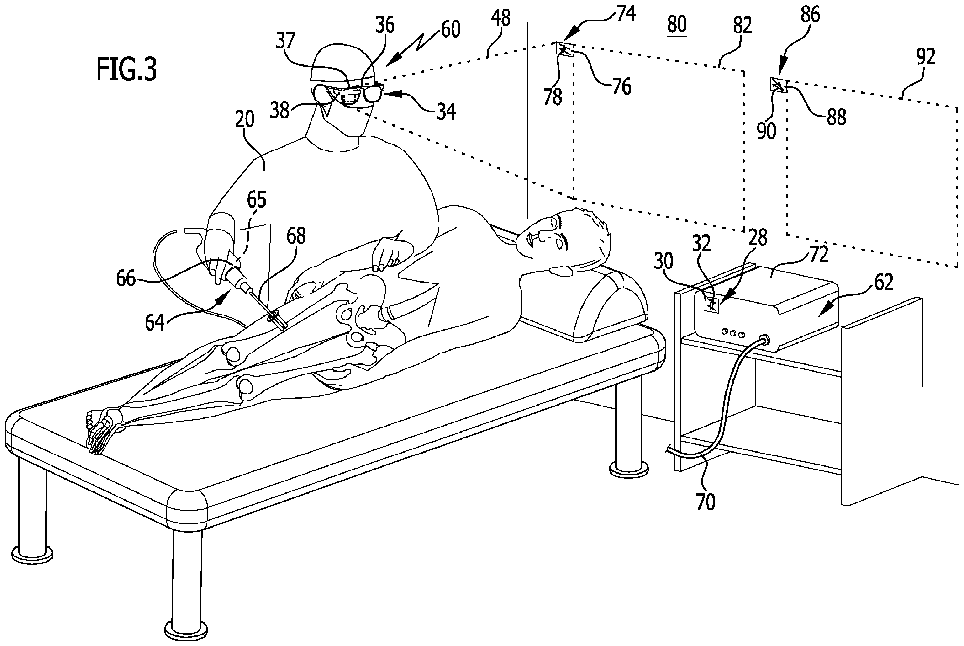

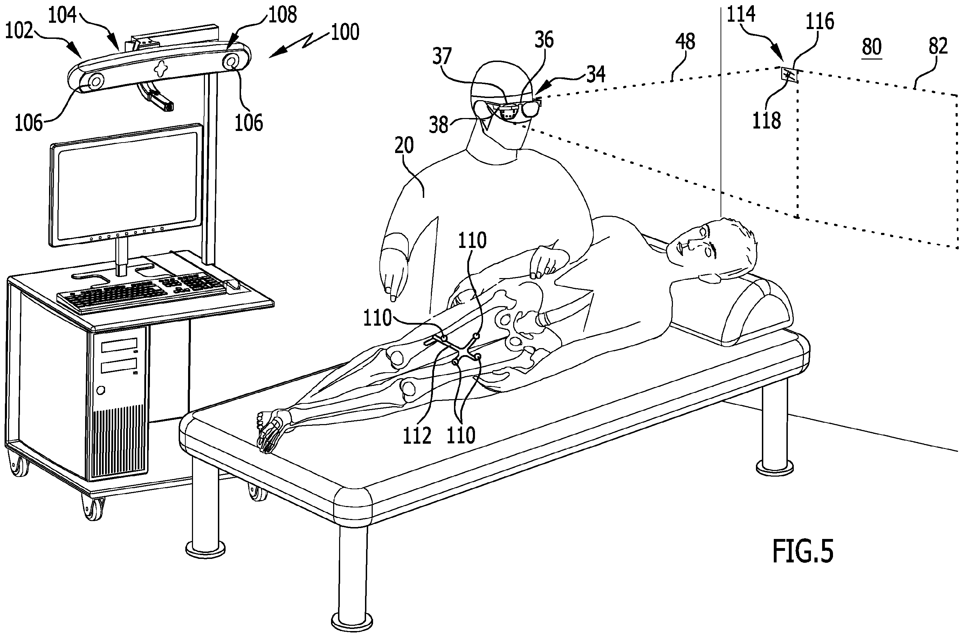

Nachfolgend wird auf in den Figuren 3 und 5 dargestellte vorteilhafte Ausführungsformen eines erfindungsgemäßen Systems eingegangen. Die mit dem System 10 erzielbaren Vorteile können unter Einsatz der nachfolgend erläuterten Systeme ebenfalls erzielt werden. Zur Vermeidung von Wiederholungen kann daher auch auf die voranstehenden Ausführungen verwiesen werden.

Für gleiche oder gleichwirkende Merkmale und Bauteile der Komponenten werden identische Bezugszeichen benutzt.

Bei dem in Figur 3 dargestellten System 60 kommt eine Vorrichtung 62 zum Einsatz, die vorliegend eine bildgebende und insbesondere eine endoskopische Vorrichtung ist. Die Vorrichtung 62 ist ebenfalls batteriebetrieben.

Die Vorrichtung 62 umfasst ein Endoskop 64 mit einer Kamera 65, die beispielsweise einen in einem Griffelement 66 angeordneten Bildsensor aufweist. Das Endoskop 64 umfasst einen in den Körper des Patienten einführbaren Schaft 68. Über eine Anschlussleitung 70 ist das Endoskop 64 mit einem Steuergerät 72 verbunden. Am Steuergerät 72 ist optional das Objekt 28 angeordnet, ausgestaltet als Markierelement 30 mit dem Code 32.

Das System 60 umfasst ein Objekt 74, das ebenfalls als Markierelement 76 mit einem Code 78 ausgestaltet ist. Die Codes 78 und 32 stimmen überein, wenn beide Markierelemente 30, 76 vorgesehen sind. Das Objekt 74 ist der Vorrichtung 62 zugeordnet.

Vorliegend ist das Markierelement 76 an einer festen oder beweglichen Wand 80 im Operationssaal festgelegt. Die Wand 80 definiert eine virtuelle Leinwand oder einen virtuellen Monitor, die bzw. der schematisch durch eine gepunktete Umrandung 82 angedeutet ist. Es kann vorgesehen sein, dass auf der Wand 80 tatsächlich eine Umrandung 82 in physischer Form angebracht ist.

Die Vorrichtung 62 kann von einem ersten in einen zweiten Zustand und insbesondere von einem Ausschaltzustand in einen Einschaltzustand überführt werden, wenn das Markierelement 76 von der Kamera 40 erfasst und identifiziert wird und der Vorrichtung 62 von der Erfassungseinrichtung 38 über die Steuereinrichtung 42 ein diesbezügliches Signal übermittelt wird.

Im eingeschalteten Zustand wird von der Kamera 65 der endoskopischen Vorrichtung 62 ein Bild bereitgestellt. Informationen betreffend den Bildinhalt werden von der Vorrichtung 62 kabellos, insbesondere per Funk, an die Datenbrille 34 und insbesondere deren Steuereinrichtung 42 übertragen. An der Bildanzeige der Anzeigeeinrichtung 46 wird das vom Endoskop 64 bereitgestellte Bild angezeigt. Dies ist in Figur 4 schematisch dargestellt, wobei dem Operateur 20 ein im Sichtfeld der Kamera 65 des Endoskops 64 liegender Gegenstand 84 im Körper des Patienten an der Anzeigeeinrichtung 46 dargestellt wird.

Die Wand 80 wirkt wie erwähnt als virtuelle Leinwand oder virtueller Monitor für den Operateur 20. Durch Betrachten der Wand 80 gewinnt der Operateur 20 den Eindruck, dass der Gegenstand 84 tatsächlich an der Wand 80 dargestellt wird. Dies ist durch gestrichelte Darstellung des Gegenstandes 84 innerhalb der Umrandung 82 in Figur 4 symbolisiert.

Die Vorrichtung 62 bleibt beispielsweise solange im zweiten Zustand (Einschaltzustand), wie das Markierelement 76 von der Kamera 40 erfasst wird. Wendet der Operateur 20 seinen Blick weg vom Markierelement 76, so dass dieses außerhalb des Erfassungsbereiches 48 liegt, wird die Vorrichtung 62 wieder in den ersten Zustand (Ausschaltzustand) überführt. Außerdem entfällt die Anzeige des Gegenstandes 84 über die Anzeigeeinrichtung 46.

Es besteht daher beim System 60 insbesondere die Möglichkeit, den Bildinhalt in der Bildanzeige der Anzeigeeinrichtung 46 abhängig vom ersten und vom zweiten Zustand darzustellen. Der jeweilige Bildinhalt ist mit dem Zustand der Vorrichtung 62 verknüpft, wobei der Bildinhalt ferner von dem von der Kamera 65 des Endoskops 64 erfassten Gegenstand abhängt.

Beim System 60 besteht, sofern das Markierelement 30 ebenfalls vorhanden ist, darüber hinaus die Möglichkeit, die Vorrichtung 62 zwischen den unterschiedlichen Zuständen zu überführen, wenn das Markierelement 30 von der Kamera 40 erfasst wird.

Das System 60 kann ein weiteres Objekt 86, ausgestaltet als Markierelement 88 mit einem Code 90, aufweisen. Das Markierelement 88 ist vorliegend ebenfalls an der Wand 80 festgelegt und definiert eine virtuelle Leinwand oder einen virtuellen Monitor, der mit einer strichpunktierten Umrandung 92 symbolisiert wird.

Durch Erfassen des Markierelementes 88 mit der Kamera 40 kann an der Bildanzeige der Anzeigeeinrichtung 46 ein Bildinhalt erkennbar für den Operateur 20 eingeblendet werden. Dieser Bildinhalt kann vom Operateur 20 als an dem virtuellen Monitor oder der virtuellen Leinwand dargestellt angesehen werden. Die Anzeigeeinrichtung 46 kann daher eine Vorrichtung 94 des Systems ausbilden, die von einem ersten Zustand (vorliegend Ausschaltzustand) in einen zweiten Zustand (vorliegend Einschaltzustand) überführbar ist und umgekehrt. Die Einblendung des Bildinhaltes entfällt, wenn der Operateur 20 seinen Blick vom Markierelement 88 so abwendet, dass dieses nicht mehr von der Kamera 40 erfassbar ist.

Ein in Figur 5 schematisch dargestelltes System 100 umfasst eine Vorrichtung 102, vorliegend in Gestalt eines chirurgischen Navigationssystems 104. Die Vorrichtung 102 kann batteriebetrieben sein.

Das Navigationssystem 104 umfasst mindestens ein und vorliegend zwei Leuchtelemente 106 sowie ferner eine Stereokamera 108. Licht, das von den Leuchtelementen 106 emittiert wird, kann von Markierelementen 110 einer chirurgischen Markiereinrichtung 112 reflektiert und von der Stereokamera 108 erfasst werden. Dies gibt die Möglichkeit, die Lage und die Orientierung der Markiereinrichtung 112 zu erkennen und ein damit referenziertes Körperteil des Patienten zu verfolgen.

Der Vorrichtung 102 ist ein Objekt 114 zugeordnet, ausgestaltet als Markierelement 116 mit einem Code 118. Wird das Markierelement 116 im Erfassungsbereich 48 von der Kamera 40 erfasst, kann die Vorrichtung 102 von einem ersten Zustand in einen zweiten Zustand überführt werden. Dabei werden insbesondere die Leuchtelemente 106 von einem Ausschaltzustand in einen Einschaltzustand überführt, um Licht zu emittieren. Darüber hinaus wird die Stereokamera 108 von einem Ausschaltzustand in einen Einschaltzustand überführt. Das von den Markierelementen 110 reflektierte Licht kann detektiert und von der Stereokamera 108 eine Bildinformation bereitgestellt werden.

An der Bildanzeige der Anzeigeeinrichtung 46 kann dem Operateur 20 wie am Beispiel des Systems 60 erläutert ein Bildinhalt angezeigt werden, der auf der Bildinformation der Stereokamera 108 beruht. Für den Operateur 20 scheint der Bildinhalt an dem virtuellen Monitor oder der virtuellen Leinwand angezeigt zu sein.

Die Vorrichtung 102 kann beispielsweise solange den zweiten Zustand einnehmen, wie das Markierelement 116 von der Kamera 40 erfasst wird. Entfällt diese Erfassung, kann die Vorrichtung 102 wieder von dem zweiten Zustand in den ersten Zustand überführt werden. Die Leuchtelemente 106 und die Stereokamera 108 können den Ausschaltzustand einnehmen.

### Bezugszeichenliste:

- 10: System
- 12: Vorrichtung
- 14: Bearbeitungswerkzeug
- 16: Bohrer
- 18: Antrieb
- 20: Operateur
- 22: Griffelement
- 24: Steuergerät
- 26: elektrische Leitung
- 28: Objekt
- 30: Markierelement
- 32: Code
- 34: Datenbrille
- 36: Brillengestell
- 37: Trageinrichtung
- 38: Erfassungseinrichtung
- 40: Kamera
- 42: Steuereinrichtung
- 44: Batterie
- 46: Anzeigeeinrichtung
- 48: Erfassungsbereich
- 50: Anzeigeelement
- 60: System
- 62: Vorrichtung
- 64: Endoskop
- 65: Kamera
- 66: Griffelement
- 68: Schaft
- 70: Anschlussleitung
- 72: Steuergerät
- 74: Objekt
- 76: Markierelement
- 78: Code
- 80: Wand
- 82: Umrandung
- 84: Gegenstand
- 86: Objekt
- 88: Markierelement
- 90: Code
- 92: Umrandung
- 94: Vorrichtung
- 100: System
- 102: Vorrichtung
- 104: Navigationssystem
- 106: Leuchtelement
- 108: Stereokamera
- 110: Markierelement
- 112: Markiereinrichtung
- 114: Objekt
- 116: Markierelement
- 118: Code

## Patentansprüche

1. System zum Ändern des Betriebszustandes mindestens einer elektrisch betriebenen Vorrichtung, insbesondere einer medizintechnischen Vorrichtung, umfassend mindestens eine von einem ersten Zustand in einen zweiten Zustand überführbare Vorrichtung (12; 62; 94; 102), mindestens ein Objekt (28; 74; 86; 114), das durch die mindestens eine Vorrichtung (12; 62; 94; 102) gebildet ist oder dieser zugeordnet ist, eine am Körper einer Bedienperson (20) tragbare Trageeinrichtung (37) und eine an dieser gehaltene Erfassungseinrichtung (38), wobei die Trageinrichtung (37) am Körper der Bedienperson (20) derart anordenbar ist, dass die Erfassungseinrichtung (38) durch Bewegung der Bedienperson (20) auf das mindestens eine Objekt (28; 74; 86; 114) ausgerichtet ist und das mindestens eine Objekt (28; 74; 86; 114) in einem Erfassungsbereich (48) der Erfassungseinrichtung (38) positioniert ist und von dieser erfasst und identifiziert wird, wobei die Erfassungseinrichtung (38) an die mindestens eine Vorrichtung (12; 62; 94; 102) bei identifiziertem Objekt (28; 74; 86; 114) ein diesbezügliches Signal derart übermittelt, dass die mindestens eine Vorrichtung (12; 62; 94; 102) vom ersten Zustand in den zweiten Zustand überführt wird, wobei die mindestens eine Vorrichtung (12; 62; 94; 102) nur dann vom ersten Zustand in den zweiten Zustand oder umgekehrt überführt wird, wenn das mindestens eine Objekt (28; 74; 86; 114) für eine vorgegebene oder vorgebbare Zeitdauer von der Erfassungseinrichtung (38) erfasst wird.

2. System nach Anspruch 1, **dadurch gekennzeichnet, dass** die mindestens eine Vorrichtung (12; 62; 94; 102) eine batteriebetriebene Vorrichtung (12; 62; 94; 102) ist.

3. System nach Anspruch 1 oder 2, **dadurch gekennzeichnet,**
- **dass** der erste Zustand ein Ausschaltzustand der mindestens einen Vorrichtung (12; 62; 94; 102) ist und der zweite Zustand ein Einschaltzustand der mindestens einen Vorrichtung (12; 62; 94; 102) oder umgekehrt; oder
- **dass** der erste Zustand ein erster Betriebszustand der mindestens einen Vorrichtung (12; 62; 94; 102) ist und der zweite Zustand ein zweiter Betriebszustand der mindestens einen Vorrichtung (12; 62; 94; 102) in einem eingeschalteten Zustand derselben.

4. System nach einem der voranstehenden Ansprüche, **dadurch gekennzeichnet, dass** die Erfassungseinrichtung (38) optisch ausgestaltet ist und eine Kamera (40) zum Erfassen des mindestens einen Objektes (28; 74; 86, 114) ist oder umfasst und/oder dass die Erfassungseinrichtung (38) kabellos mit der mindestens einen Vorrichtung (12; 62; 94; 102) in Wirkverbindung steht, insbesondere über eine Funkverbindung.

5. System nach einem der voranstehenden Ansprüche, **dadurch gekennzeichnet, dass** an die Trageinrichtung (37) mindestens eine Batterie (44) zur Energieversorgung der mindestens einen Erfassungseinrichtung (38) gehalten ist.

6. System nach einem der voranstehenden Ansprüche, **dadurch gekennzeichnet, dass** die Trageinrichtung (37) am Kopf der Bedienperson (20) anordenbar ist.

7. System nach einem der voranstehenden Ansprüche, **dadurch gekennzeichnet, dass** die Trageinrichtung (37) eine Brille oder ein Brillengestell (36) ist oder umfasst, insbesondere dass die Trageinrichtung (37) mit der Erfassungseinrichtung (38) als Datenbrille (34) ausgestaltet ist, und/oder dass die Trageinrichtung ein Stirnband oder eine Haube ist oder umfasst.

8. System nach einem der voranstehenden Ansprüche, **dadurch gekennzeichnet,**
- **dass** die Vorrichtung (12; 62; 94; 102) den zweiten Zustand solange einnimmt, wie das mindestens eine Objekt (28; 74; 86; 114) von der Erfassungseinrichtung (38) erfasst wird, und anderenfalls den ersten Zustand einnimmt; oder
- **dass** die Vorrichtung (12; 62; 94; 102) nach der Einnahme des zweiten Zustandes in diesem verbleibt und vom zweiten Zustand in den ersten Zustand überführbar ist, wenn das mindestens eine Objekt (28; 74; 86; 114) von der Erfassungseinrichtung (38) erneut erfasst und der mindestens einen Vorrichtung (12; 62; 94; 102) ein diesbezügliches Signal übermittelt wird.

9. System nach einem der voranstehenden Ansprüche, **dadurch gekennzeichnet,**
- **dass** das mindestens eine Objekt (28; 74; 86; 114) ein Markierelement (30; 76; 88; 116) ist, insbesondere dass das Markierelement (30) an der mindestens einen Vorrichtung (12; 62) festgelegt ist; oder
- **dass** als das mindestens eine Objekt die mindestens eine Vorrichtung von der Erfassungseinrichtung optisch erfassbar ist zum Erkennen der mindestens einen Vorrichtung anhand von deren charakteristischen Merkmalen, insbesondere Form und/oder Farbe.

10. System nach einem der voranstehenden Ansprüche, **dadurch gekennzeichnet, dass** zumindest eines der Folgenden vorgesehen ist:
- eine Vorrichtung (12) mit mindestens einem antreibbaren chirurgischen Bearbeitungswerkzeug (14), wobei der erste Zustand und der zweite Zustand mit dem Antrieb (18) des Bearbeitungswerkzeuges (14) verknüpft sind;
- eine Vorrichtung (102) mit mindestens einem Leuchtelement (106), wobei der erste Zustand und der zweite Zustand mit dem Leuchtzustand des Leuchtelementes (106) verknüpft sind;
- eine bildgebende Vorrichtung (62; 102) mit mindestens einer Kamera (65; 108), wobei der erste Zustand und der zweite Zustand mit einer Bereitstellung eines Bildes der Kamera (108) verknüpft sind, und wobei die bildgebende Vorrichtung (62) insbesondere eine endoskopische Vorrichtung (62) ist.

11. System nach einem der voranstehenden Ansprüche, **dadurch gekennzeichnet, dass** die mindestens eine Vorrichtung (62; 94) eine Anzeigeeinrichtung (46) mit einer Bildanzeige aufweist, an der ein Bildinhalt betreffend den Betrieb der mindestens einen Vorrichtung (62; 94) darstellbar ist, insbesondere dass an der Bildanzeige ein Bildinhalt abhängig vom ersten Zustand und/oder zweiten Zustand darstellbar ist.

12. System nach einem der voranstehenden Ansprüche, **dadurch gekennzeichnet, dass** als Vorrichtung (94) eine Anzeigeeinrichtung (46) mit einer Bildanzeige vorgesehen ist, wobei der erste Zustand und der zweite Zustand mit einem Bildinhalt der Bildanzeige verknüpft sind.

13. System nach Anspruch 12, **dadurch gekennzeichnet, dass** die Anzeigeeinrichtung (46) an der Trageinrichtung (37) gehalten ist, insbesondere dass eine Datenbrille (34) mit Anzeigeeinrichtung (46) zur Einblendung des Bildinhaltes für die Bedienperson (20) vorhanden ist.

## Claims

1. System for changing the operating state of at least one electrically operated apparatus, in particular a medical-technical apparatus, comprising at least one apparatus (12; 62; 94; 102) that is transferable from a first state into a second state, at least one object (28; 74; 86; 114) that is formed by the at least one apparatus (12; 62; 94; 102) or is associated therewith, a support device (37) that is wearable on the body of an operator (20), and a detection device (38) that is held on said support device (37), wherein the support device (37) is arrangeable on the body of the operator (20) in such a way that by movement of the operator (20), the detection device (38) is aligned to the at least one object (28; 74; 86; 114) and the at least one object (28; 74; 86; 114) is positioned in a detection region (48) of the detection device (38) and is detected and identified thereby, wherein when the object (28; 74; 86; 114) is identified, the detection device (38) transmits a signal relating thereto to the at least one apparatus (12; 62; 94; 102) in such a way that the at least one apparatus (12; 62; 94; 102) is transferred from the first state into the second state, wherein the at least one apparatus (12; 62; 94; 102) is transferred from the first state into the second state or vice versa only when the at least one object (28; 74; 86; 114) is detected by the detection device (38) for a predetermined or predeterminable duration.

2. System in accordance with Claim 1, **characterized in that** the at least one apparatus (12; 62; 94; 102) is a battery-operated apparatus (12; 62; 94; 102).

3. System in accordance with Claim 1 or 2, **characterized in that**
- the first state is a switched-off state of the at least one apparatus (12; 62; 94; 102) and the second state is a switched-on state of the at least one apparatus (12; 62; 94; 102) or vice versa; or
- the first state is a first operating state of the at least one apparatus (12; 62; 94; 102) and the second state is a second operating state of the at least one apparatus (12; 62; 94; 102) in a switched-on state thereof.

4. System in accordance with any one of the preceding Claims, **characterized in that** the detection device (38) is of optical configuration and is or comprises a camera (40) for detecting the at least one object (28; 74; 86, 114) and/or **in that** the detection device (38) is wirelessly in operative connection with the at least one apparatus (12; 62; 94; 102), in particular by way of a radio connection.

5. System in accordance with any one of the preceding Claims, **characterized in that** at least one battery (44) for supplying energy to the at least one detection device (38) is held on the support device (37).

6. System in accordance with any one of the preceding Claims, **characterized in that** the support device (37) is arrangeable on the head of the operator (20).

7. System in accordance with any one of the preceding Claims, **characterized in that** the support device (37) is or comprises a pair of glasses or a glasses frame (36), in particular **in that** the support device (37) with the detection device (38) is configured as data glasses (34), and/or **in that** the support device is or comprises a headband or a hood.

8. System in accordance with any one of the preceding Claims, **characterized in that**
- the apparatus (12; 62; 94; 102) adopts the second state as long as the at least one object (28; 74; 86; 114) is being detected by the detection device (38), and otherwise adopts the first state; or
- the apparatus (12; 62; 94; 102), after adopting the second state, remains therein and is transferable from the second state into the first state when the at least one object (28; 74; 86; 114) is detected again by the detection device (38) and a signal relating thereto is transmitted to the at least one apparatus (12; 62; 94; 102).

9. System in accordance with any one of the preceding Claims, **characterized in that**
- the at least one object (28; 74; 86; 114) is a marking element (30; 76; 88; 116), in particular **in that** the marking element (30) is fixed to the at least one apparatus (12; 62); or
- the at least one apparatus is optically detectable as the at least one object by the detection device for identifying the at least one apparatus on the basis of its characteristic features, in particular shape and/or color.

10. System in accordance with any one of the preceding Claims, **characterized in that** at least one of the following is provided:
- an apparatus (12) with at least one drivable surgical treatment tool (14), wherein the first state and the second state are linked to the drive (18) of the treatment tool (14);
- an apparatus (102) with at least one luminous element (106), wherein the first state and the second state are linked to the luminous state of the luminous element (106);
- an imaging apparatus (62; 102) with at least one camera (65; 108), wherein the first state and the second state are linked to a provision of an image from the camera (108), and wherein the imaging apparatus (62) is, in particular, an endoscopic apparatus (62).

11. System in accordance with any one of the preceding Claims, **characterized in that**, the at least one apparatus (62; 94) has a display device (46) with an image display on which an image content relating to the operation of the at least one apparatus (62; 94) is displayable, in particular **in that** an image content is displayable on the image display in dependence on the first state and/or second state.

12. System in accordance with any one of the preceding Claims, **characterized in that** a display device (46) with an image display is provided as an apparatus (94), wherein the first state and the second state are linked to an image content of the image display.

13. System in accordance with Claim 12, **characterized in that** the display device (46) is held on the support device (37), in particular **in that** data glasses (34) with a display device (46) for superimposing the image content for the operator (20) are present.

## Revendications

1. Système pour modifier l'état de fonctionnement d'au moins un appareil actionné électriquement, en particulier un appareil technique médical, comprenant au moins un appareil (12; 62; 94; 102) pouvant être amené d'un premier état à un deuxième état, au moins un objet (28; 74; 86; 114), qui est formé par le au moins un appareil (12; 62; 94; 102) ou qui lui est associé, un dispositif de port (37) qui peut être porté sur le corps d'un opérateur (20) et un dispositif de détection (38) maintenu sur celui-ci, où le dispositif de port (37) peut être disposé sur le corps de l'opérateur (20) de telle manière que le dispositif de détection (38) est aligné avec le au moins un objet (28; 74; 86; 114) par le mouvement de l'opérateur (20) et le au moins un objet (28; 74; 86; 114) est positionné dans une zone de détection (48) du dispositif de détection (38) et est détecté et identifié par ce dernier, où le dispositif de détection (38) transmet au au moins un appareil (12; 62; 94; 102) lorsque l'objet (28; 74; 86; 114) est identifié un signal concernant celui-ci, de telle sorte que le au moins un appareil (12; 62; 94; 102) est amené à passer du premier état au deuxième état, où le au moins un appareil (12; 62; 94; 102) n'est amené à passer du premier état au deuxième état ou inversement que lorsque le au moins un objet (28; 74; 86; 114) est détecté par le dispositif de détection (38) pendant une durée prédéterminée ou pouvant être prédéterminée.

2. Système selon la revendication 1, **caractérisé en ce que** le au moins un appareil (12; 62; 94; 102) est un appareil (12; 62; 94; 102) alimenté par batterie.

3. Système selon la revendication 1 ou 2, **caractérisé**
- **en ce que** le premier état est un état désactivé du au moins un appareil (12; 62; 94; 102) et le deuxième état est un état activé du au moins un appareil (12; 62; 94; 102) ou inversement; ou
- **en ce que** le premier état est un premier état de fonctionnement du au moins un appareil (12; 62; 94; 102) et le deuxième état est un deuxième état de fonctionnement du au moins un appareil (12; 62; 94; 102) dans un état activé de celui-ci.

4. Système selon l'une des revendications précédentes, **caractérisé en ce que** le dispositif de détection (38) est conçu optiquement et est ou comprend une caméra (40) pour détecter le au moins un objet (28; 74; 86, 114) et/ou **en ce que** le dispositif de détection (38) est en liaison fonctionnelle sans fil avec le au moins un appareil (12; 62; 94; 102), en particulier par le biais d'une liaison radio.

5. Système selon l'une des revendications précédentes, **caractérisé en ce qu'**au moins une batterie (44) pour fournir de l'énergie au au moins un dispositif de détection (38) est maintenue sur le dispositif de port (37).

6. Système selon l'une des revendications précédentes, **caractérisé en ce que** le dispositif de port (37) peut être disposé sur la tête de l'opérateur (20).

7. Système selon l'une des revendications précédentes, **caractérisé en ce que** le dispositif de port (37) est ou comprend des lunettes ou des montures de lunettes (36), en particulier **en ce que** le dispositif de port (37) avec le dispositif de détection (38) est conçu sous forme de lunettes de données (34),
et/ou **en ce que** le dispositif de port est ou comprend un bandeau ou un bonnet.

8. Système selon l'une des revendications précédentes, **caractérisé**
- **en ce que** l'appareil (12; 62; 94; 102) revêt le deuxième état tant que le au moins un objet (28; 74; 86; 114) est détecté par le dispositif de détection (38), et revêt sinon le premier état; ou
- **en ce que** l'appareil (12; 62; 94; 102) après avoir revêtu le deuxième état demeure dans celui-ci et peut être amené à passer du deuxième état au premier état quand le au moins un objet (28; 74; 86; 114) est à nouveau détecté par le dispositif de détection (38) et un signal correspondant est transmis au au moins un appareil (12; 62; 94; 102).

9. Système selon l'une des revendications précédentes, **caractérisé**
- **en ce que** le au moins un objet (28; 74; 86; 114) est un élément de marquage (30; 76; 88; 116), en particulier en ce que l'élément de marquage (30) est fixé sur le au moins un appareil (12; 62); ou
- **en ce que**, en tant que le au moins un objet, le au moins un appareil peut être détecté optiquement par le dispositif de détection afin de reconnaître le au moins un appareil à l'aide de ses caractéristiques, en particulier sa forme et/ou sa couleur.

10. Système selon l'une des revendications précédentes, **caractérisé en ce qu'**au moins l'un des suivants est prévu:
- un appareil (12) avec au moins un outil de traitement chirurgical (14) pouvant être entraîné, où le premier état et le deuxième état sont liés à l'entraînement (18) de l'outil de traitement (14);
- un appareil (102) avec au moins un élément lumineux (106), où le premier état et le deuxième état sont liés à l'état lumineux de l'élément lumineux (106);
- un appareil d'imagerie (62; 102) avec au moins une caméra (65; 108), où le premier état et le deuxième état sont liés à la fourniture d'une image de la caméra (108), et où l'appareil d'imagerie (62) est en particulier un appareil endoscopique (62).

11. Système selon l'une des revendications précédentes, **caractérisé en ce que** le au moins un appareil (62; 94) comporte un dispositif d'affichage (46) avec un affichage d'image sur lequel un contenu d'image concernant le fonctionnement du au moins un appareil (62; 94) peut être représenté, en particulier **en ce qu'**un contenu d'image peut être représenté sur l'affichage d'image en fonction du premier état et/ou du deuxième état.

12. Système selon l'une des revendications précédentes, **caractérisé en ce qu'**un dispositif d'affichage (46) avec un affichage d'image est prévu comme appareil (94), où le premier état et le deuxième état sont liés à un contenu d'image de l'affichage d'image.

13. Système selon la revendication 12, **caractérisé en ce que** le dispositif d'affichage (46) est maintenu sur le dispositif de port (37), en particulier **en ce que** des lunettes de données (34) avec un dispositif d'affichage (46) pour insérer le contenu d'image pour l'opérateur (20) sont présentes.
